# EUROPÄISCHE PATENTSCHRIFT

(11) **EP 2 362 102 B1**
(45) Veröffentlichungstag und Bekanntmachung des Hinweises auf die Patenterteilung: **03.10.2012**
(21) Anmeldenummer: 10001644.3
(22) Anmeldetag: 18.02.2010
(51) Int. Cl.: F04B 49/06, F04B 53/06, F04B 11/00

(54) **Dosierpumpenaggregat**
Metering pump aggregate
Agrégat de pompes de dosage

(43) Veröffentlichungstag der Anmeldung: 31.08.2011
(73) Patentinhaber: Grundfos Management A/S, 8850 Bjerringbro (DK)
(72) Erfinder: Gerz, Sergei, 76327 Pfinztal (DE); Simon, Markus, 75335 Dobel (DE)
(74) Vertreter: Hemmer, Arnd

(56) Entgegenhaltungen:
- EP-A1- 1 757 809
- WO-A1-01/83989
- WO-A1-85/01993
- WO-A1-2006/108606
- US-A- 4 131 393

## Beschreibung

Die Erfindung betrifft ein Dosierpumpenaggregat gemäß dem Oberbegriff des Anspruchs 1, sowie ein Verfahren zum Ansteuern eines Antriebsmotors eines solchen Dosierpumpenaggregates.

Dosierpumpenaggregate sind bekannterweise als Verdrängerpumpen ausgebildet, welche einen Dosierraum mit einem Verdrängerkörper beispielsweise in Form einer Membran aufweisen. Wenn ausgasende Medien gefördert werden, besteht die Problematik, dass sich im Dosierraum Gasblasen bilden können. Dies gilt insbesondere auch beim Stillstand der Pumpe. Diese Gasblasen beeinträchtigen die Dosiergenauigkeit, da sich die Gasblasen einen Teil des zu dosierenden Volumens der Flüssigkeit einnehmen und darüber hinaus kompressibel sind. Insofern ist es erstrebenswert, derartige Gasblasen möglichst schnell aus dem Dosierraum zu entfernen. Dies gelingt jedoch nicht immer und insbesondere bei Stillstand der Pumpe kann es zur Ansammlung von größeren Gasmengen im Dosierraum kommen. Bei einem bestimmten Luft-Flüssigkeit-Verhältnis kann es dann zu einem Blockieren der Pumpe kommen, dass heißt die Dosierpumpe kann keine Flüssigkeit mehr ansaugen.

WO 01/83989 A1 offenbart eine Pumpe mit den merkmalen der oberbegriffs des anspruchs 1 zum Pumpen verflüssigten Gases, bei welcher die Pumpgeschwindigkeit während des Hubes verändert werden kann. So ist vorgesehen, die Geschwindigkeit in der Mitte des Hubes zu erhöhen und zum Ende hin wieder abzusenken, um die Menge des pro Hubes geförderten verflüssigten Gases exakt steuern zu können.

US 4,131,393 offenbart eine Flüssigkeitspumpe zum konstanten Fördern von Flüssigkeiten mit einem geringeren Pulsieren des Druckes. Dazu ist vorgesehen, den Saughub schneller als den Druckhub durchzuführen.

Dieser Stand der Technik zeigt jedoch keine Lösung, einen möglichen Ausfall aufgrund einer größeren Ansammlung von Gasblasen im Dosierraum zu verhindern.

Es ist Aufgabe der Erfindung, ein verbessertes Dosierpumpenaggregat bereitzustellen, bei welchem ein Ausfall aufgrund einer größeren Ansammlung von Gasblasen im Dosierraum verhindert werden kann.

Diese Aufgabe wird durch ein Dosierpumpenaggregat mit den in Anspruch 1 angegebenen Merkmalen, sowie durch ein Verfahren zur Steuerung des Verdrängerantriebs eines Dosierpumpenaggregates mit den in Anspruch 17 angegebenen Merkmalen gelöst. Bevorzugte Ausführungsformen ergeben sich aus den Unteransprüchen, der nachfolgenden Beschreibung sowie den Figuren.

Das erfindungsgemäße Dosierpumpenaggregat weist in bekannter Weise einen Dosierraum mit einem an diesen angrenzenden bzw. in diesem angeordneten Verdrängerkörper auf. Der Verdrängerkörper ist durch einen Verdrängerantrieb, z. B. einen Antriebsmotor, bewegbar. Zum Ansteuern bzw. Regeln des Verdrängerantriebs ist darüber hinaus eine Steuereinrichtung vorgesehen, welche beispielsweise Geschwindigkeit bzw. Drehzahl des Verdrängerantriebs einstellt bzw. steuert. Ferner ist das Dosierpumpenaggregat bevorzugt mit einem Erkennungssystem zum Erkennen von Gasblasen im Dosierraum versehen. Ein solches System kann beispielsweise im Pumpen- bzw. Dosierkopf angeordnete Sensoren aufweisen und ist beispielsweise aus WO 2006/108606A1 bekannt.

Erfindungsgemäß ist die Steuereinrichtung derart ausgebildet, dass sie in zumindest einem Betriebszustand, z. B. bei Erkennen von Gasblasen im Dosierraum durch das Erkennungssystem den Verdröngerantrieb in spezieller Weise ansteuert. Diese spezielle Ansteuerung kann z. B. bei einem bestimmten Volumenanteil von Gasblasen am Hubvolumen von der Steuereinrichtung gewählt werden. Diese Ansteuerung könnte jedoch auch immer verwendet werden. Die Steuereinrichtung ist dazu so ausgebildet bzw. weist ein entsprechendes Programm zur Ansteuerung des Verdrängerantriebs auf, gemäß dem ein Druckhub des Verdrängerkörpers mit einer ersten geringeren Hubgeschwindigkeit begonnen wird und im weiteren Verlauf des Druckhubes die Hubgeschwindigkeit auf eine zweite erhöhte Hubgeschwindigkeit erhöht wird. Durch eine höhere Hubgeschwindigkeit am Ende des Hubes, welche durch eine höhere Geschwindigkeit des Verdrängerantriebs, z. B. eine höhere Drehzahl des Antriebsmotors erreicht wird, wird z. B. bewirkt, dass aufgrund des dadurch erzeugten Druckimpulses im Dosierraum das Druckventil, d.h. das Rückschlagventil auf der Druckseite, verspätet schließt, so dass zu Beginn eines nachfolgenden Saughubes eine Flüssigkeits-Rückströmung aus der Druckleitung in den Dosierkopf stattfindet. Dadurch wird eine Verbesserung des Flüssigkeit-Luft Verhältnisses im Dosierraum erreicht. Das heißt der Flüssigkeitsanteil im Dosierraum wird erhöht. Auf diese Weise wird eine höhere Sicherheit gegen einen Dosierausfall augrund eines zu hohen Luftanteils (Airlocking) erreicht. Durch die erhöhte Hubgeschwindigkeit wird darüber hinaus ein schnelleres Ausdrücken der Gasblasen aus dem Dosierraum z. B. gegen Ende des Hubes erreicht.

Ausgehend von der ersten geringeren Hubgeschwindigkeit kann die Geschwindigkeit auch in mehreren Stufen auf eine zweite, gegebenenfalls dritte und weitere erhöhte Hubgeschwindigkeiten gesteigert werden. Dabei kann die Geschwindigkeitserhöhung sprunghaft oder auch über eine Rampe erfolgen.

Auch wenn die Steuereinrichtung bevorzugt derart ausgebildet ist, dass sie die vorangehend und nachfolgend beschriebene spezielle Ansteuerung bzw. Ansteuercharakteristik für einen Druckhub wählt, so ist jedoch zu verstehen, dass es auch möglich ist, diese beschriebene spezielle Ansteuerung im Saughub einzusetzen, d. h. die Hubgeschwindigkeit z. B. gegen Ende des Saughubes zu erhöhen.

Auch kann diese spezielle Ansteuerung automatisch aufgrund des Erkennens von Gasblasen durch die Steuereinrichtung aktiviert werden oder es könnte eine manuelle Aktivierbarkeit vorgesehen sein. Auch könnte eine solche Ansteuerung immer beim Betrieb des Dosierpumpenaggregates von der Steuereinrichtung durchgeführt werden. Bevorzugt ist die Steuereinrichtung derart ausgebildet, dass sie den Verdrängerantrieb bei der speziellen Antriebsstrategie z. B. nach Erkennen von Gasblasen im Dosierraum so ansteuert, dass die Hubgeschwindigkeit gegen Ende des Hubes bzw. Druckhubes erhöht wird. Das bedeutet, dass der Großteil des Hubes mit einer oder mehreren geringeren Hubgeschwindigkeiten erfolgt und die genannte höhere Druckgeschwindigkeit erst in einem vergleichbar kleinen Endbereich des Hubes zum Einsatz kommt, so dass insgesamt kein höherer Volumenstrom als ein Soll-Volumenstrom gefördert wird.

Gemäß einer weiteren bevorzugten Ausführungsform ist die Steuereinrichtung derart ausgebildet, dass sie den Verdrängerantrieb z. B. bei Erkennen von Gasblasen im Dosierraum derart ansteuert, dass ein auf den Druckhub folgender Saughub mit einer erhöhten, vorzugsweise der zweiten Hubgeschwindigkeit begonnen wird. Dadurch wird die Rückströmung aus dem Druckkanal noch einmal begünstigt.

Bevorzugt macht der Teil des Hubes mit der zweiten erhöhten Hubgeschwindigkeit, das heißt bevorzugt der letzte Teil des Hubes, zwischen 2 und 15 % des gesamten Hubes aus. Der Gesamtförderstrom über den gesamten Hub, insbesondere Druckhub betrachtet erhöht sich damit durch die schnellere Hubgeschwindigkeit bzw. Antriebsdrehzahl des Antriebsmotors zu Ende des Hubes nur geringfügig. Dabei ist zu verstehen, dass der Hub, von welchem ein Anteil zwischen 2 und 15 % mit der erhöhten Hubgeschwindigkeit gefahren wird, kein maximal möglicher Hub des Dosierpumpenaggregates sein muss. Es kann sich hierbei vielmehr auch um einen verringerten Hub handeln, von welchem wiederum ein Anteil mit der erhöhten Hubgeschwindigkeit erfolgt.

Weiter bevorzugt ist die Steuereinrichtung derart ausgebildet, dass sie die erste geringere Hubgeschwindigkeit bei der Steuerung nach Erkennen von Gasblasen so einstellt, dass unter Berücksichtung des Volumens der Gasblasen durch den Hub des Verdrängerkörpers ein gewünschter Soll-Förderstrom der zu fördernden Flüssigkeit erreicht werden kann. Das heißt insgesamt erfolgt der Antrieb für ein höheres Fördervolumen, um das Volumen und die Kompressibilität der Gasblasen auszugleichen und sicherzustellen, dass im Wesentlichen der gewünschte Soll-Förderstrom der zu fördernden Flüssigkeit erreicht werden kann.

Die erste Hubgeschwindigkeit, wird gemäß einer weiteren bevorzugten Ausführungsform derart optimiert, dass Gasblasen im Dosierraum ausreichend Zeit verbleibt, im Dosierraum in den Druckkanal aufzusteigen, so dass die Gasblasen möglichst vollständig aus dem Dosierraum verdrängt bzw. hinausgefördert werden. Dazu sollte die Hubgeschwindigkeit nicht zu hoch eingestellt sein. Auch dies kann in einem entsprechenden Steuerprogramm der Steuereinrichtung zur Verwendung beim Erkennen von Gasblasen oder einer Menge von Gasblasen über einem vorbestimmten Grenzwert berücksichtigt werden.

Die erste geringere Hubgeschwindigkeit ist bevorzugt kleiner oder gleich hundert Hübe pro Minute, wobei bei Förderung von Schleichmengen die Geschwindigkeit beispielsweise auch kleiner als zwanzig Hübe pro Minute sein kann. Die zweite erhöhte Hubgeschwindigkeit liegt vorzugsweise zwischen einhundertzwanzig und dreihundert Hüben pro Minute.

Weiter bevorzugt ist die Steuereinrichtung derart ausgebildet, dass sie die erste geringere Hubgeschwindigkeit, die zweite erhöhte Hubgeschwindigkeit und den Anteil des Teilhubes mit der zweiten Hubgeschwindigkeit am gesamten Hub, insbesondere Druckhub derart einstellt, dass über den gesamten Hub ein durchschnittlicher Förderstrom erreicht werden kann, welcher einem Soll-Förderstrom entspricht. Das heißt die erhöhte Hubgeschwindigkeit im zweiten Teil bzw. am Ende des Hubes mit einem damit verbundenen erhöhten Förderstrom wird durch einen entsprechend geringeren Förderstrom aufgrund einer geringeren Hubgeschwindigkeit bzw. Antriebsdrehzahl im ersten Teil des Hubes ausgeglichen. Durch die Verringerung der Antriebsdrehzahl bzw. der Hubgeschwindigkeit im ersten Teil des Druckhubes wird darüber hinaus, wie vorangehend beschreiben, das Entfernen der Gasblasen aus dem Dosierraum begünstigt.

Das Erkennungssystem zum Erkennen von Gasblasen im Dosierraum weist bevorzugt zumindest einen Drucksensor zum Erfassen des Druckes der von der Dosierpumpe geförderten Flüssigkeit auf und ist derart ausgestaltet, dass es die Anwesenheit von Gasblasen anhand der von dem zumindest einen Drucksensor erfassten Druckwerte erkennt. Dazu können die Druckwerte mit Sollwerten verglichen werden. Dabei kann gleichzeitig die von der Steuereinrichtung eingestellte Geschwindigkeit des Verdrängerantriebs zur Auswahl entsprechender Soll-Druckwerte beispielsweise aus einem Speicher im Erkennungssystem herangezogen werden. Das Erkennungssystem kann als eigenständige elektronische Einheit ausgebildet sein, jedoch auch mit der Steuereinrichtung eine integrierte elektronische Einrichtung bilden. Insbesondere kann das Erkennungssystem abgesehen von der erforderlichen Hardware wie Sensoren als Software in einer Steuereinrichtung realisiert sein.

Weiter bevorzugt ist der zumindest eine Drucksensor im oder am Dosierraum oder einem sich vom Dosierraum stromabwärts erstreckenden Druckkanal angeordnet. Das bedeutet, der Sensor ist bevorzugt möglichst nah am Dosierraum angeordnet, um dort unverfälscht von äußeren Einflüssen in einem sich anschließenden hydraulischen System den Förderdruck der Pumpe zu erfassen. Darüber hinaus wird es möglich, den Sensor in das Dosierpumpenaggregat und insbesondere einen Pumpenkopf des Dosierpumpenaggregates zu integrieren, so dass es nicht erforderlich ist, bei der Montage des Dosierpumpenaggregates einen externen Sensor zu montieren und mit dem Dosierpumpenaggregat bzw. der Steuereinrichtung oder dem Erkennungssystem zur Signalübertragung zu verbinden. Die erforderlichen Systeme bzw. Leitungen zur Signalübertragung können so auch in das Dosierpumpenaggregat geschützt integriert werden.

Gemäß einer weiteren bevorzugten Ausführungsform ist das Erkennungssystem derart ausgebildet, dass es den Druckverlauf über zumindest einen Druckhub des Verdrängerkörpers erfasst und anhand des Druckverlaufes die Anwesenheit von Gasblasen erkennt. Dabei kann der Druckverlauf vorzugsweise ebenfalls mit einem vorgegebenen Solldruckverlauf verglichen werden. Im Falle, dass Gasblasen im Dosierraum vorhanden sind, steigt der Druck in der Regel aufgrund der Kompressibilität des Gases erst mit einer gewissen Verzögerung zum Beginn des Druckhubes an. So kann beispielsweise zur Erkennung von Gasblasen von dem Erkennungssystem erfasst werden, ob der Druckverlauf direkt nach Beginn des Druckhubes ansteigt oder mit einer gewissen Verzögerung. Liegt diese Verzögerung über einem vorbestimmten Grenzwert, kann darauf auf das Vorhandensein einer gewissen Gasmenge im Dosierraum geschlossen werden. Darüber hinaus wird es auch möglich, aus der Verzögerung bzw. dem Druckverlauf auf die Quantität des Gases im Dosierraum zu schließen.

Daher ist das Erkennungssystem bevorzugt derart ausgebildet, dass es den Anteil der Gasblasen am Gesamthubvolumen bestimmt. So kann das oben beschriebene spezielle Antriebs- bzw. Fahrprogramm zur Ansteuerung des Verdrängerantriebs bei Erkennen von Gasblasen von der Steuereinrichtung beispielsweise dann gewählt werden, wenn ein bestimmter Grenzwert für den Anteil von Gasblasen am Gesamtvolumen des Dosierraumes erkannt wird. Der Grenzwert ist vorzugsweise so gewählt, dass unterhalb des Grenzwertes ein Förderausfall sicher vermieden werden kann und dann bei Erreichen des Grenzwertes durch die spezielle Antriebsstrategie, welche von der Steuereinrichtung dann gewählt wird, ebenfalls vermieden werden kann.

Somit ist die Steuereinrichtung bevorzugt so ausgebildet, dass sie bei Erkennen von Gasblasen mit einem Anteil am Hubvolumen des, welcher größer als ein vorbestimmter erster Grenzwert ist, den Verdrängerantrieb derart ansteuert, dass ein Druckhub des Verdrängerkörpers mit einer ersten geringeren Hubgeschwindigkeit begonnen wird und im weiteren Verlauf des Hubes die Hubgeschwindigkeit auf eine zweite erhöhte Hubgeschwindigkeit erhöht wird. Der Grenzwert ist so gewählt, dass bei geringeren Anteilen von Gas am Volumen des Dosierraumes das übliche Programm zur Steuerung des Antriebes von der Steuereinrichtung ausgeführt wird und nicht das oben beschriebene spezielle Programm bzw. die oben beschriebene spezielle Antriebsstrategie. Dabei liegt der Grenzwert bei einem Gasanteil am Hubvolumen, welcher derart gering ist, dass es nicht zu einem Förderausfall der Pumpe kommen kann. Der Grenzwert wird so gewählt, dass ab einem Gasanteil, bei welchem ein Förderausfall zu befürchten ist, die oben beschriebene spezielle Antriebsstrategie gewählt wird, bei welcher die Hubgeschwindigkeit zum Ende des Hubes hin erhöht wird.

Bevorzugt entspricht der erste Grenzwert einem Volumenanteil > 65 %, vorzugsweise einem Volumenanteil von 70 oder 75 % des Hubvolumens. Das bedeutet, wenn das Gasvolumen weniger als 65 %, 70 % bzw. 75 % des Hubvolumens ausmacht, wird noch nicht die spezielle Antriebsstrategie gewählt, erst wenn ein derart hoher Gasanteil erfasst wird, wird insbesondere im Druckhub die Hubgeschwindigkeit bzw. die Antriebsgeschwindigkeit des Verdrängerantriebs zum Hubende hin erhöht.

Weiter ist es bevorzugt, dass die Steuereinrichtung derart ausgebildet ist, dass sie den Verdrängerantrieb solange derart ansteuert, dass die Druckhübe des Verdrängerkörpers mit einer ersten geringeren Hubgeschwindigkeit begonnen werden und im weiteren Verlauf des Hubes die Hubgeschwindigkeit auf eine zweite erhöhte Hubgeschwindigkeit erhöht wird, bis von den Erkennungssystem erkannt wird, dass die Gasblasen einen Anteil am Hubvolumen haben, welcher kleiner als ein vorbestimmter zweiter Grenzwert ist. Auf diese Weise wird sichergestellt, dass die Dosierpumpe solange in dem Modus mit der erhöhten Hubgeschwindigkeit zum Ende des Hubes hin betrieben wird, bis wieder eine derartige Betriebssicherheit gewährleistet ist, bei welcher ein Förder- bzw. Dosierausfall nicht mehr zu befürchten ist. Der vorbestimmte zweite Grenzwert ist vorzugsweise kleiner als der erste Grenzwert, um ein wiederholtes Pendeln zwischen den beiden Betriebmodi zu verhindern.

Gemäß einer besonders bevorzugten Ausführungsform ist der Verdrängerantrieb ein Antriebsmotor, insbesondere ein Schrittmotor. Über einen solchen Schrittmotor lassen sich die Hübe des Verdrängerkörpers sehr genau steuern, da der Antriebsmotor in einer Vielzahl von Einzelschritten drehend bewegt wird. Über die Anzahl der Einzelschritte pro Zeiteinheit können die Drehzahl und damit auch die Hubgeschwindigkeit variiert werden. Darüber hinaus ist es auch möglich die Hublänge durch Drehen des Antriebsmotors um eine bestimmte Schrittzahl genau zu definieren. Es können jedoch auch andere drehende Antriebsmotoren, beispielsweise EC-Motoren oder Servomotoren zum Einsatz kommen. Bei drehenden Antriebsmotoren ist ein Getriebe vorgesehen, welches die Drehbewegung in eine Linearbewegung umsetzt, beispielsweise ein Exzenter, ein Nockentrieb, Kurbeltrieb oder Schneckentrieb. Anstatt eines drehenden Antriebsmotors kann jedoch auch ein elektrischer Linearantrieb oder Linearmotor Verwendung finden.

Die Erfindung betrifft ferner ein Verfahren zum Steuern eines Verdrängerantriebs eines Dosierpumpenaggregates. Dabei handelt es sich bevorzugt um ein Dosierpumpenaggregat gemäß der vorangehenden Beschreibung. Das Verfahren ist so ausgebildet, dass insbesondere bei Erkennen von Gasblasen im Dosierraum, vorzugsweise bei Erkennen eines bestimmten Anteils von Gasblasen am Gesamthubvolumen, ein Verdrängerantrieb zum Bewegen eines Verdröngerkörpers der Dosierpumpe derart angesteuert wird, dass ein Druckhub des Verdrängerkörpers mit einer ersten geringen Hubgeschwindigkeit begonnen wird und im weiteren Verlauf des Hubes die Hubgeschwindigkeit auf eine zweite erhöhte Hubgeschwindigkeit erhöht wird. Dieses Erhöhen erfolgt vorzugsweise zum Ende des Hubes bzw. Druckhubes hin, so dass der Großteil des Hubes mit einer ersten geringeren Hubgeschwindigkeit erfolgt. Die Erhöhung von der ersten geringeren Hubgeschwindigkeit auf die zweite erhöhte Hubgeschwindigkeit kann sprunghaft oder auch kontinuierlich oder in mehreren Schritten ansteigend erfolgen. Hierzu kann die vorangehend beschriebene Steuereinrichtung entsprechend ausgestaltet sein. Im Übrigen wird das Verfahren bevorzugt so ausgeführt, wie oben anhand des Dosierpumpenaggregates beschrieben.

Nachfolgend wird die Erfindung beispielhaft anhand der beigefügten Figuren beschrieben. In diesen zeigt:

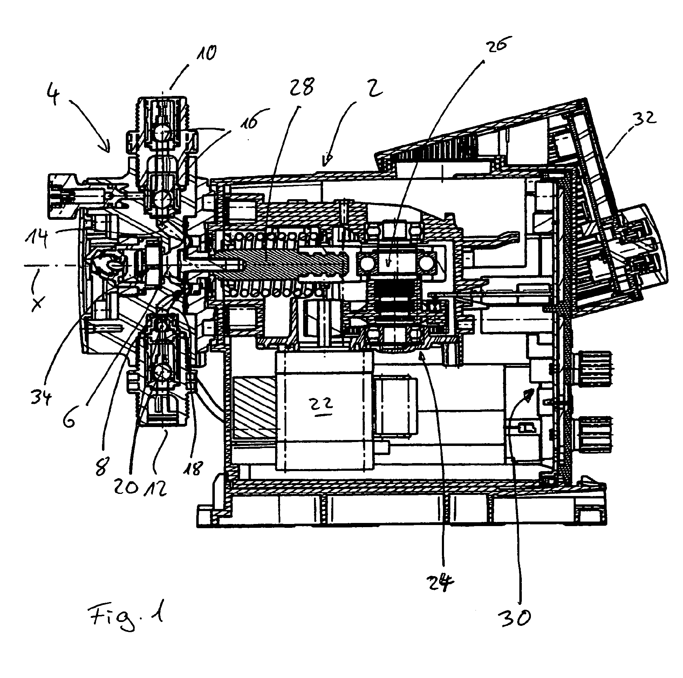

- Figur 1 -: eine Schnittansicht durch ein erfindungsgemäße Dosierpumpenaggregat und
- Figur 2 -: ein Diagramm, in welchem die Drehzahl des Antriebsmotors über einen Druckhub aufgetragen ist.

Das gezeigte Pumpenaggregat weist ein Antriebsgehäuse 2 mit einem stirnseitig daran angesetzten Pumpenkopf 4 auf. In dem Pumpenkopf 4 ist eine Dosierkammer 6 ausgebildet, welche an einer Seite von einer Membran 8 begrenzt wird. Die Membran 8 ist linear beweglich entlang der Hubachse X. Der Dosierraum 6 ist mit einem Druckanschluss 10 und einem Sauganschluss 12 verbunden. In dem Druckkanal 14, welcher den Dosierraum 6 mit dem Druckanschluss 10 verbindet, sind zwei Rückschlagventile 16 angeordnet. In dem Saugkanal 18, welcher den Dosierraum 6 mit dem Sauganschluss 12 verbindet, sind zwei Rückschlagventile 20 angeordnet.

In dem Antriebsgehäuse 2 ist ein Verdrängerantrieb in Form eines elektrischen Antriebsmotors 22, vorzugsweise in Form eines Schrittmotors, angeordnet. Der Antriebsmotor 22 treibt über ein Getriebe 24 und einen Exzenter 26 ein Pleuel 28 an, welches die Membran 8 in linearer Hubbewegung entlang der Hubachse X versetzt. Zur Steuerung des Antriebsmotors 22 ist im Antriebsgehäuse 2 eine Steuereinrichtung bzw. Steuerelektronik 30 angeordnet. Die Steuerelektronik 30 steuert den Antriebsmotor 22 nach Drehzahl und Drehwinkel in Abhängigkeit vorgegebener Parameter. Diese können beispielsweise über eine Bedieneinheit 32 eingestellt werden. Die Steuerelektronik 30 beinhaltet darüber hinaus ein Erkennungssystem zum Erkennen von Gasblasen in dem Dosierraum 6. Dieses Erkennungssystem kann in der Steuerelektronik 30 als Hardware- und/oder Software realisiert sein. Das Erkennungssystem beinhaltet einen Drucksensor 34, welcher den Druck im Dosierraum 6 erfasst und entsprechende Ausgangssignale über eine hier nicht gezeigte Leitungsverbindung an die Steuerelektronik 36 weitergibt.

Anhand des Druckverlaufes im Druckhub kann über den Drucksensor 34 erkannt werden, ob Gasblasen im Dosierraum vorhanden sind. In dem Fall, dass Gasblasen im Dosierraum 6 vorhanden sind, steigt der Druck zu Beginn des Druckhubes verzögert an, da zunächst das Gas in den Gasblasen komprimiert wird. Die Dauer dieser Verzögerung des Druckanstieges ist repräsentativ für den Anteil des Gasvolumens am Hubvolumen, so dass von der Steuerelektronik 30 bzw. dem darin realisierten Erkennungssystem auch die Quantität des Gasanteil am Hubvolumen erfasst werden kann.

Wenn nun ein vorbestimmter Gasanteil von diesem Erkennungssystem erfasst wird, beispielsweise ein Gasanteil von mehr als 70 % des Hubvolumens besteht die Gefahr eines Förder- bzw. Dosierausfalles, das heißt die Dosierpumpe würde im Saughub keine weitere zu fördernde Flüssigkeit ansaugen, stattdessen würde durch Hub der Membran 8 lediglich das Gasvolumen komprimiert und expandiert. Um dies zu verhindern wird bei einem vorgegebenen Gasanteil von beispielsweise 70 % von der Steuerelektronik ein bestimmtes Steuerverfahren bzw. eine bestimmte Steuerstrategie zur Steuerung des Antriebsmotors 22 umgesetzt.

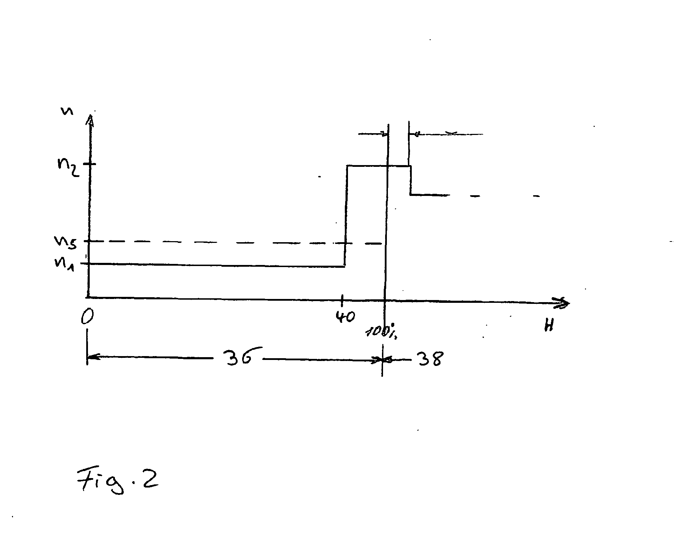

Diese Steuerstrategie bedeutet, dass der Antriebsmotor 22 in diesem Beispiel zu Beginn des Druckhubes zunächst mit einer geringeren Drehzahl n₁ betrieben wird, wie in Figur 2 gezeigt ist. Die Drehzahl des Antriebsmotors 22 ist proportional zur Hubgeschwindigkeit der Membran 8. In Figur 2 ist die Motordrehzahl n über dem Hub H aufgetragen. Dabei ist im Detail ein Druckhub 36 gezeigt, an den sich dann ein nicht mehr vollständig dargestellter Saughub 38 darstellt. Der Druckhub 36 wird mit einer verringerten Drehzahl n₁ des Antriebsmotors 22 begonnen, so dass entsprechend die Membran 8 zunächst mit einer niedrigeren Hubgeschwindigkeit verfährt. Am Ende des Druckhubes 36 wird aus Punkt 40 die Drehzahl von der niedrigeren Drehzahl n₁ auf eine erhöhte Drehzahl n₂ erhöht. Damit erhöht sich auch die Hubgeschwindigkeit auf eine proportionale erhöhte Hubgeschwindigkeit. Mit dieser erhöhten Drehzahl n₂ wird der letzte Teil des Druckhubes 36, beispielsweise die letzen 2 - 20 % des Druckhubes ausgeführt. Auch der Beginn des Saughubes 38 wird mit dieser erhöhten Drehzahl n₂ ausgeführt, anschließend kann die Drehzahl im Saughub wieder reduziert werden. Durch die erhöhte Drehzahl n₂ und die damit verbundene erhöhte Hubgeschwindigkeit zum Ende des Druckhubes 36 wird erreicht, dass zum Ende des Druckhubes in dem Dosierraum 6 ein erhöhter Druckimpuls erzeugt wird, welcher dann zu Beginn des Saughubes 38 das Schließen des Rückschlagventils 16 verzögert. Auf diese Weise wird erreicht, dass es zu Beginn des Saughubes zu einer Rückströmung von Fluid aus dem Teil des Druckkanals 14, welcher stromabwärts des ersten, das heißt dem Dosierraum 6 zugewandten, Rückschlagventiles 16 gelegen ist, kommt. Dadurch erhöht sich im Dosierraum 6 der Flüssigkeitsanteil und ein Gas-Flüssigkeits-Verhältnis im Dosierraum wird entsprechend reduziert, wodurch die Betriebssicherheit gegen einen Förderausfall erhöht wird, da sich der Gasanteil entsprechend am Gesamtvolumen verringert.

Die niedrige Drehzahl n₁ sowie die erhöhte Drehzahl n₂ und der Anteil des Druckhubes zwischen dem Punkt 40 und dem Ende des Druckhubes 36, welcher mit der erhöhten Antriebsdrehzahl n₂ ausgeführt wird, werden so gewählt, dass im Durchschnitt des gesamten Druckhubes ein Soll-Förderstrom erreicht werden kann, welcher sonst üblicherweise mit einer Soll-Drehzahl nₛ erreicht würde. Dabei ist nₛ ist die Solldrehzahl, mit welcher der Antriebsmotor 22 im Normalzustand zum Erreichen eines Soll-Förderstromes betrieben würde. Beim Ausführen der beschriebenen Antriebsstrategie zum Sicherstellen der Förderung wird entsprechend die niedrigere Drehzahl n₁ geringer als die Solldrehzahl nₛ und die erhöhte Drehzahl n₂ höher als die Solldrehzahl nₛ gewählt, so dass im Durchschnitt über den gesamten Druckhub 36 eine Solldrehzahl nₛ und damit ein verbundener Soll-Förderstrom erzielt wird. Die Verringerung der Drehzahl zu Beginn des Druckhubes mit der damit verbundenen geringeren Hubgeschwindigkeit hat darüber hinaus den Vorteil, dass bei geringerer Hubgeschwindigkeit Gasblasen im Dosierraum 6 mehr Zeit verbleibt, im Dosierraum in den Druckkanal 14 aufzusteigen, so dass Gasblasen zusätzlich besser aus dem Dosierraum 6 hinausgefördert werden können.

Die anhand von Figur 2 beschriebene Antriebstrategie wird von der Steuerelektronik 30 solange aufrechterhalten, bis das Erkennungssystem über den Drucksensor 34 einen Gasanteil am Volumen des Dosierraumes 6 erfasst, welcher unter einem vorbestimmten Grenzwert liegt. Dieser Grenzwert ist vorzugsweise niedriger als der Grenzwert, bei welchem die beschriebene Antriebsstrategie begonnen wird. Das heißt ggf. wird diese Antriebsstrategie für mehrere Druckhübe 36 in dieser Weise ausgeführt.

### Bezugszeichenliste

- 2 -: Antriebsgehäuse
- 4 -: Pumpenkopf
- 6 -: Dosierraum
- 8 -: Membran
- 10 -: Druckanschluss
- 12 -: Sauganschluss
- 14 -: Druckkanal
- 16 -: Rückschlagventil
- 18 -: Saugkanal
- 20 -: Rückschlagventil
- 22 -: Antriebsmotor
- 24 -: Getriebe
- 26 -: Exzenter
- 28 -: Pleuel
- 30 -: Steuerelektronik
- 32 -: Bedieneinheit
- 34 -: Drucksensor
- 36 -: Druckhub
- 38 -: Saughub
- 40 -: Punkt des Druckhubes, an welchem die Drehzahl verändert wird
- n -: Drehzahl
- n₁ -: niedrigere Drehzahl
- n₂ -: höhere Drehzahl
- ns -: Solldrehzahl
- H -: Hub
- X -: Hubachse

## Patentansprüche

1. Dosierpumpenaggregat mit einem Dosierraum (6), einem diesen begrenzenden, durch einen Verdrängerantrieb (22) bewegbaren Verdrängerkörper sowie einer Steuereinrichtung zum Ansteuern des Verdrängerantriebs (22), **dadurch gekennzeichnet, dass** die Steuereinrichtung (30) derart ausgebildet ist, dass sie zumindest in einem bestimmten Betriebszustand den Verdrängerantrieb (22) derart ansteuert, dass ein Druckhub (36) des Verdrängerkörpers mit einer ersten geringeren Hubgeschwindigkeit begonnen wird und im weiteren Verlauf des Druckhubes (36) die Hubgeschwindigkeit auf eine zweite erhöhte Hubgeschwindigkeit am Ende des Druckhubes (36) erhöht wird.

2. Dosierpumpenaggregat nach Anspruch 1, **dadurch gekennzeichnet, dass** die Steuereinrichtung derart ausgestaltet ist, dass sie zumindest in einem bestimmten Betriebszustand den Verdrängerantrieb (22) derart ansteuert, dass ein Druckhub (36) des Verdrängerkörpers mit einer ersten geringeren Hubgeschwindigkeit begonnen wird und im weiteren Verlauf des Druckhubes (36) die Hubgeschwindigkeit auf eine zweite erhöhte Hubgeschwindigkeit erhöht wird.

3. Dosierpumpenaggregat nach Anspruch 1 oder 2, **dadurch gekennzeichnet, dass** die Steuereinrichtung (30) mit einem Erkennungssystem zum Erkennen von Gasblasen im Dosierraum (6) versehen ist, wobei die Steuereinrichtung derart ausgebildet ist, dass sie bei Erkennen von Gasblasen im Dosierraum (6) den Verdrängerantrieb (22) derart ansteuert, dass ein Hub (36) des Verdrängerkörpers mit einer ersten geringeren Hubgeschwindigkeit begonnen wird und im weiteren Verlauf des Hubes (36) die Hubgeschwindigkeit auf eine zweite erhöhte Hubgeschwindigkeit erhöht wird.

4. Dosierpumpenaggregat nach einem der vorangehenden Ansprüche, **dadurch gekennzeichnet, dass** die Steuereinrichtung (30) derart ausgebildet ist, dass sie den Verdröngerantrieb (22) derart ansteuert, dass die Hubgeschwindigkeit gegen Ende des Hubes (36) erhöht wird

5. Dosierpumpenaggregat nach einem der vorangehenden Ansprüche, **dadurch gekennzeichnet, dass** die Steuereinrichtung (30) derart ausgebildet ist, dass sie den Verdrangerantrieb (22) derart ansteuert, dass ein auf einen Druckhub (36) folgender Saughub (38) mit einer erhöhten, vorzugsweise der zweiten Hubgeschwindigkeit begonnen wird.

6. Dosierpumpenaggregat nach einem der vorangehenden Ansprüche, **dadurch gekennzeichnet, dass** der Teil des Druckhubes (36) mit der zweiten erhöhten Hubgeschwindigkeit zwischen 2 und 15% des gesamten Hubes (36) ausmacht.

7. Dosierpumpenaggregat nach einem der vorangehenden Ansprüche, **dadurch gekennzeichnet, dass** die Steuereinrichtung (30) derart ausgebildet ist, dass sie die erste geringere Hubgeschwindigkeit so einstellt, dass unter Berücksichtigung des Volumens der Gasblasen durch den Hub (H) des Verdrängerkörpers ein gewünschter Soll-Förderstrom der zu fördernden Flüssigkeit erreicht werden kann.

8. Dosierpumpenaggregat nach einem der vorangehenden Ansprüche, **dadurch gekennzeichnet, dass** die Steuereinrichtung (30) derart ausgebildet ist, dass sie die erste geringere Hubgeschwindigkeit, die zweite erhöhte Hubgeschwindigkeit und den Anteil des Teilhubes mit der zweiten Hubgeschwindigkeit am gesamten Druckhub (36) derart einstellt, dass über den gesamten Hub (H) ein durchschnittlicher Förderstrom erreicht werden kann, welcher einem Soll-Förderstrom entspricht.

9. Dosierpumpenaggregat nach einem der Ansprüche 3 bis 8, **dadurch gekennzeichnet, dass** das Erkennungssystem zumindest einen Drucksensor (34) zum Erfassen des Druckes der von der Dosierpumpe geförderten Flüssigkeit aufweist und derart ausgestaltet ist, dass es die Anwesenheit von Gasblasen anhand der von dem zumindest einen Drucksensor (34) erfassten Druckwerte erkennt.

10. Dosierpumpenaggregat nach Anspruch 9, **dadurch gekennzeichnet, dass** der zumindest eine Drucksensor (34) im oder am Dosierraum (6) oder einem sich vom Dosierraum (6) stromabwärts erstreckenden Druckkanal (14) angeordnet ist.

11. Dosierpumpenaggregat nach Anspruch 9 oder 10, **dadurch gekennzeichnet, dass** das Erkennungssystem derart ausgebildet ist, dass es den Druckverlauf über zumindest einen Druckhub (36) des Verdrängerkörpers erfasst und anhand des Druckverlaufes die Anwesenheit von Gasblasen erkennt.

12. Dosierpumpenaggregat nach einem der Ansprüche 3 bis 11, **dadurch gekennzeichnet, dass** das Erkennungssystem derart ausgebildet ist, dass es den Anteil der Gasblasen am Gesamtvolumen des Dosierraumes (6) bestimmt.

13. Dosierpumpenaggregat nach Anspruch 12, **dadurch gekennzeichnet, dass** die Steuereinrichtung (30) derart ausgebildet ist, dass sie bei Erkennen von Gasblasen mit einem Anteil am Volumen des Dosierraumes (6), welcher größer als ein vorbestimmter erster Grenzwert ist, den Verdröngerantrieb (22) derart ansteuert, dass ein Hub (36) des Verdrängerkörpers mit einer ersten geringeren Hubgeschwindigkeit begonnen wird und im weiteren Verlauf des Hubes (36) die Hubgeschwindigkeit auf eine zweite erhöhte Hubgeschwindigkeit erhöht wird.

14. Dosierpumpenaggregat nach Anspruch 13, **dadurch gekennzeichnet, dass** der erste Grenzwert einem Volumenanteil größer als 65 %, vorzugsweise von 70 oder 75 % des Hubvolumens entspricht.

15. Dosierpumpenaggregat nach Anspruch 13 oder 14, **dadurch gekennzeichnet, dass** die Steuereinrichtung (30) derart ausgebildet ist, dass sie den Verdrängerantrieb (22) solange derart ansteuert, dass die Hübe (36) des Verdrängerkörpers mit einer ersten geringeren Hubgeschwindigkeit begonnen werden und im weiteren Verlauf des Hubes (36) die Hubgeschwindigkeit auf eine zweite erhöhte Hubgeschwindigkeit erhöht wird, bis von dem Erkennungssystem erkannt wird, dass die Gasblasen einen Anteil am Volumen des Dosierraumes (6) haben, welcher kleiner als ein vorbestimmter zweiter Grenzwert ist.

16. Dosierpumpenaggregat nach einem der vorangehenden Ansprüche, **dadurch gekennzeichnet, dass** der Verdrängerantrieb (22) ein Schrittmotor ist.

17. Verfahren zum Ansteuern eines Verdrängerantriebs (22) eines Dosierpumpenaggregates **dadurch gekennzeichnet, dass** ein Verdrängerantrieb (22) zum Bewegen eines Verdrängerkörpers derart angesteuert wird, dass ein Druckhub (36) des Verdrängerkörpers mit einer ersten geringeren Hubgeschwindigkeit begonnen wird und im weiteren Verlauf des Druckhubes (36) die Hubgeschwindigkeit auf eine zweite erhöhte Hubgeschwindigkeit am ende des druckhubes erhöht wird.

## Claims

1. A metering pump aggregate with a metering chamber (6), a positive-displacement body that adjoins the latter and can be moved by a positive-displacement drive (22), as well as a controller for actuating the positive-displacement drive (22), **characterized in that** the controller (30) is designed to actuate the positive-displacement drive (22) at least in a specific operating state in such a way that a pressure stroke (36) of the positive-displacement body is started at a first, lower stroke rate, and the stroke rate is increased to a second, elevated stroke rate at the end of the pressure stroke (36) as the pressure stroke (36) continues.

2. The metering pump aggregate according to claim 1, **characterized in that** the controller is designed to actuate the positive-displacement drive (22) at least in a specific operating state in such a way that a pressure stroke (36) of the positive-displacement body is started at a first, lower stroke rate, and the stroke rate is increased to a second, elevated stroke rate as the pressure stroke (36) continues.

3. The metering pump aggregate according to claim 1 or 2, **characterized in that** the controller (30) is provided with a detection system for detecting gas bubbles in the metering chamber (6), wherein the controller is designed to actuate the positive-displacement drive (22) in such a way after detecting gas bubbles in the metering chamber (6) that a stroke (36) of the positive-displacement body is started at a first, lower stroke rate, and the stroke rate is increased to a second, elevated stroke rate as the stroke (36) continues.

4. The metering pump aggregate according to one of the preceding claims, **characterized in that** the controller (30) is designed to actuate the positive-displacement drive (22) in such a way that the stroke rate is increased toward the end of the stroke (36).

5. The metering pump aggregate according to one of the preceding claims, **characterized in that** the controller (30) is designed to actuate the positive-displacement drive (22) in such a way that an intake stroke (38) following a pressure stroke (36) is started at an elevated, preferably the second, stroke rate.

6. The metering pump aggregate according to one of the preceding claims, **characterized in that** the portion of the pressure stroke (36) at the second elevated stroke rate comprises between 2 and 15% of the entire stroke (36).

7. The metering pump aggregate according to one of the preceding claims, **characterized in that** the controller (30) is designed to set the first, lower stroke rate in such a way that a desired setpoint conveyed flow of the liquid to be conveyed can be achieved via the stroke (H) of the positive-displacement body, taking into account the volume of the gas bubbles.

8. The metering pump aggregate according to one of the preceding claims, **characterized in that** the controller (30) is designed to set the first, lower stroke rate, the second, elevated stroke rate, and the percentage of the partial stroke at the second stroke rate in the overall pressure stroke (36) in such a way that an average conveyed flow corresponding to the setpoint conveyed flow can be achieved over the entire stroke (H).

9. The metering pump aggregate according to one of claims 3 to 8, **characterized in that** the detection system has at least one pressure sensor (34) for acquiring the pressure of the liquid conveyed by the metering pump, and is designed to detect the presence of gas bubbles based on the pressure values acquired by the at least one pressure sensor (34).

10. The metering pump aggregate according to claim 9, **characterized in that** the at least one pressure sensor (34) is arranged in or on the metering chamber (6) or a pressure channel (14) extending downstream from the metering chamber (6).

11. The metering pump aggregate according to claim 9 or 10, **characterized in that** the detection system is designed to acquire the pressure progression over at least one pressure stroke (36) of the positive-displacement body, and detect the presence of gas bubbles based on the pressure progression.

12. The metering pump aggregate according to one of claims 3 to 11, **characterized in that** the detection system is designed to determine the portion of gas bubbles in the overall volume of the metering chamber (6).

13. The metering pump aggregate according to claim 12, **characterized in that** the controller (30) is designed to actuate the positive-displacement drive (22) after detecting gas bubbles comprising a percentage of the volume of the metering chamber (6) that exceeds a preset first limit in such a way that a stroke (36) of the positive-displacement body is started at a first, lower stroke rate, and the stroke rate is increased to a second, elevated stroke rate as the stroke (36) continues.

14. The metering pump aggregate according to claim 13, **characterized in that** the first limit corresponds to a volumetric percentage that exceeds 65%, preferably 70 or 75%, of the stroke volume.

15. The metering pump aggregate according to claim 13 or 14, **characterized in that** the controller (30) is designed to actuate the positive-displacement drive (22) until the strokes (36) of the positive-displacement body are started at a first, lower stroke rate, and the stroke rate is increased to a second, elevated stroke rate as the stroke (36) continues, until the detection system detects that the percentage of gas bubbles in the volume of the metering chamber (6) is less than a preset second limit.

16. The metering pump aggregate according to one of the preceding claims, **characterized in that** the positive-displacement drive (22) is a stepping motor.

17. A method for actuating a positive-displacement drive (22) of a metering pump aggregate, **characterized in that** a positive-displacement drive (22) is actuated for moving a positive-displacement body in such a way that a pressure stroke (36) of the positive-displacement body is started at a first, lower stroke rate, and the stroke rate is increased to a second, elevated stroke rate at the end of the pressure stroke as the pressure stroke (36) continues.

## Revendications

1. Groupe pompe doseuse comprenant une chambre de dosage (6), un corps de déplaceur qui limite cette chambre et peut être mis en mouvement par un entraînement de déplaceur (22), ainsi qu'un dispositif de commande servant à commander l'entraînement de déplaceur (22), **caractérisé en ce que** le dispositif de commande (30) est conçu de telle manière qu'il commande l'entraînement de déplaceur (22), au moins dans un certain état de fonctionnement, de façon à ce qu'une course de compression (36) du corps de déplaceur soit commencée à une première vitesse de course, réduite, et que, dans la suite de la course de compression (36), la vitesse de course soit portée à une deuxième vitesse de course, augmentée, à la fin de la course de compression (36).

2. Groupe pompe doseuse selon la revendication 1, **caractérisé en ce que** le dispositif de commande est conçu de telle manière qu'il commande l'entraînement de déplaceur (22), au moins dans un état de fonctionnement déterminé, de façon à ce qu'une course de compression (36) du corps de déplaceur soit commencée à une première vitesse de course, réduite, et que, dans la suite de la course de compression (36), la vitesse de course soit portée à une deuxième vitesse de course, augmentée.

3. Groupe pompe doseuse selon la revendication 1 ou 2, **caractérisé en ce que** le dispositif de commande (30) est muni d'un système de détection destiné à détecter des bulles de gaz dans la chambre de dosage (6), le dispositif de commande étant conçu de telle manière qu'en cas de détection de bulles de gaz dans la chambre de dosage (6), il commande l'entraînement de déplaceur (22) de façon à ce qu'une course (36) du corps de déplaceur soit commencée à une première vitesse de course, réduite, et que, dans la suite du déroulement de la course (36), la vitesse de course soit portée à une deuxième vitesse de course, augmentée.

4. Groupe pompe doseuse selon l'une des revendications précédentes, **caractérisé en ce que** le dispositif de commande (30) est conçu de telle manière qu'il commande l'entraînement de déplaceur (22) de façon à ce que la vitesse de course soit augmentée vers la fin de la course (36).

5. Groupe pompe doseuse selon l'une des revendications précédentes, **caractérisé en ce que** le dispositif de commande (30) est conçu de telle manière qu'il commande l'entraînement de déplaceur (22) de façon à ce qu'une course d'aspiration (38) qui fait suite à une course de compression (36) soit commencée à une vitesse de course augmentée, de préférence à la deuxième vitesse de course.

6. Groupe pompe doseuse selon l'une des revendications précédentes, **caractérisé en ce que** la partie de la course de compression (36) exécutée à la deuxième vitesse de course, augmentée, représente entre 2 et 15 % de la course complète (36).

7. Groupe pompe doseuse selon l'une des revendications précédentes, **caractérisé en ce que** le dispositif de commande (30) est conçu de telle manière qu'il règle la première vitesse de course, réduite, de façon à ce qu'en tenant compte du volume des bulles de gaz, il soit possible d'atteindre par la course (H) du corps de déplaceur un débit de consigne souhaité du liquide à refouler.

8. Groupe pompe doseuse selon l'une des revendications précédentes, **caractérisé en ce que** le dispositif de commande (30) est conçu de telle manière qu'il règle la première vitesse de course, réduite, la deuxième vitesse de course, augmentée, et la fraction de la course partielle parcourue à la deuxième vitesse de course, sur l'ensemble de la course (H), de façon telle qu'il soit possible d'atteindre un débit moyen qui correspond à un débit de consigne.

9. Groupe pompe doseuse selon l'une des revendications 3 à 8, **caractérisé en ce que** le système de détection comprend au moins un capteur de pression (34) destiné à capter la pression du liquide déplacé par la pompe doseuse, et est conçu de telle manière qu'il détecte la présence de bulles de gaz en se basant sur les valeurs de pression acquises par le capteur de pression (34), au moins au nombre de un.

10. Groupe pompe doseuse selon la revendication 9, **caractérisé en ce que** le capteur de pression (34), au moins au nombre de un, est disposé dans ou au niveau de la chambre de dosage (6), ou dans ou au niveau d'un canal de pression (14) qui s'étend en aval de la chambre de dosage (6).

11. Groupe pompe doseuse selon la revendication 9 ou 10, **caractérisé en ce que** le système de détection est conçu de telle manière qu'il acquière le profil de pression sur au moins une course de compression (36) du corps de déplaceur et qu'il détecte la présence de bulles de gaz en se basant sur le profil de pression.

12. Groupe pompe doseuse selon l'une des revendications 3 à 11, **caractérisé en ce que** le système de détection est conçu de telle manière qu'il détermine la proportion des bulles de gaz dans le volume total de la chambre de dosage (6).

13. Groupe pompe doseuse selon la revendication 12, **caractérisé en ce que** le dispositif de commande (30) est conçu de telle manière qu'en cas de détection de bulles de gaz qui représentent une proportion du volume de la chambre de dosage (6) supérieure à une première valeur limite prédéterminée, il commande l'entraînement de déplaceur (22) de façon telle qu'une course (36) du corps de déplaceur soit commencée à une première vitesse de course, réduite et que, dans la suite de la course (36), la vitesse de course soit portée à une deuxième vitesse de course, augmentée.

14. Groupe pompe doseuse selon la revendication 13, **caractérisé en ce que** la première valeur limite correspond à une proportion de volume supérieure à 65 %, de préférence de 70 ou 75 % du volume de course.

15. Groupe pompe doseuse selon la revendication 13 au 14, **caractérisé en ce que** le dispositif de commande (30) est conçu de telle manière qu'il commande l'entraînement de déplaceur (22) de façon à ce que les courses (36) du corps de déplaceur soient commencées à une première vitesse de course, réduite, et que, dans la suite du déroulement de la course (36), la vitesse de course soit portée à une deuxième vitesse de course, augmentée, jusqu'à ce qu'il soit détecté, par le système de détection, que les bulles de gaz représentent une proportion du volume de la chambre de dosage (6) qui est plus petite qu'une deuxième valeur limite prédéterminée.

16. Groupe pompe doseuse selon l'une des revendications précédentes, **caractérisé en ce que** l'entraînement de déplaceur (22) est un moteur pas-à-pas.

17. Procédé pour commander un entraînement de déplaceur (22) d'un groupe pompe doseuse, **caractérisé en ce qu'**un entraînement de déplaceur (22) destiné à déplacer un corps de déplaceur est commandé de manière telle qu'une course de compression (36) du corps de déplaceur soit commencée à une première vitesse de course, réduite, et que dans la suite du déroulement de la course de compression (36), la vitesse de la course soit portée à une deuxième vitesse, augmentée, à la fin de la course de compression.
